# EUROPEAN PATENT APPLICATION

(11) **EP 4 597 502 A1**
(43) Date of publication of application: **06.08.2025**
(21) Application number: 24155540.8
(22) Date of filing: 02.02.2024
(51) Int. Cl.: G16B 10/00, G16B 20/00, G16B 40/00

(54) **ANALYSING A SET OF BIOLOGICAL SAMPLES FOR IDENTIFICATION OF MICROORGANISMS**

(71) Applicant: Biomiris Capital Group B.V., 1098 XG Amsterdam (NL)
(72) Inventor: BUDDING, Andries Edward, 1098 XG Amsterdam (NL); BUDDING, Jord Jan Anton, 1098 XG Amsterdam (NL); PORTEGIES, Cees, 1098 XG Amsterdam (NL)
(74) Representative: V.O.

(57) **Abstract**

Method of analysing a set of biological samples for identification of microorganisms potentially present in the samples, the method comprising steps of obtaining a first set of analysis data, by an electronic processor of the supervisory system, using the first set of analysis data, for each sample in the set of biological samples, determining if a subsequent analysis is required, and generating a first subset of samples with samples for which it is determined that subsequent analysis is required. The method further comprises analysing the samples in the first subset of biological samples in a second analysis arrangement. The disclosure also contemplates determining, for each sample, whether a microorganism characteristic is to be determined.

## Description

### TECHNICAL FIELD

The present disclosure relates to the field of microbial diagnostics, in particular to rapid identification of microorganisms in samples at the species or strain level, in particular bacteria in clinical and non-clinical samples.

### BACKGROUND

WO2021112673 discloses a method of identifying a microorganism in a biological sample by polymerase chain reaction. In the method, at least one microbial rRNA internal transcribed spaced region (ITS) is PCR amplified, to generate PCR amplicons. A high resolution melting curve is recorded for these PCR amplicons, and a length of the PCR amplicons is recorded, for example by capillary electrophoresis or sequencings.

To identify a microorganism present in the biological sample, the high resolution melting curve is compared with a database comprising high resolution melting curves of reference amplicons generated from reference microbial species or strains of known taxonomic identity using the same set of amplification primers, to thereby obtain a first taxonomic identity indicator of a microorganism present in said sample. Also, the length of each PCR amplicon having a distinct length is compared with a database comprising PCR amplicon lengths of reference amplicons generated from reference microbial species or strains of known taxonomic identity using the same set of amplification primers, to thereby obtain a second taxonomic identity indicator of a microorganism present in said sample.

Both the first taxonomic identity indicator and the second taxonomic identity indicator are used to identify a microorganism present in said sample to the species or strain level.

### SUMMARY

Although the method disclosed in WO2021112673 provides for an accurate and fast method of identifying microorganisms in a biological sample, it continues to be an aim to increase speed with which microorganisms can be identified in biological samples and/or to use less resources to identify microorganisms in biological samples, both in clinical and non-clinical settings. Furthermore, it may be a general aim to determine one or more microorganism characteristics in biological samples suspected of comprising one or more microorganisms.

In the context of the present disclosure, microorganisms include bacteria, viruses, fungi and parasites.

A first aspect of the present disclosure provides a method of analysing a set of biological samples. In general, the analysing of the set of biological samples aims at identifying no, one, or multiple microorganisms in each sample in the set of samples, and optionally the analysing of the set of biological samples aims at obtaining a subset of samples for which a microorganism characteristic is to be determined.

The method according to the first aspect comprises steps of:
- in a first analysis arrangement, analysing the samples in the set of biological samples, thereby obtaining a first set of analysis data;
- transferring the first set of analysis data to a supervisory system;
- by an electronic processor of the supervisory system, using the first set of analysis data, for each sample in the set of biological samples:
   a) identifying no, one, or multiple microorganisms in the sample and generating microorganism data based on the identifying;
      and at least one of:
      b1) based on the microorganism data, determining whether a subsequent identification analysis is required;
- by the electronic processor of the supervisory system, generating a first identification subset of samples with samples for which it is determined that a subsequent identification analysis is required;
- in a second analysis arrangement, analysing the samples in the first identification subset of biological samples, thereby obtaining a second set of analysis data;
- transferring the second set of analysis data to the supervisory system; and
- by the electronic processor of the supervisory system, using the second set of analysis data, for each sample in the first subset of samples:
   c1) identifying no, one, or multiple microorganisms in the sample and generating microorganism data based on the identifying; or
   d1) determining that another subsequent identification analysis is required;
      and/or
   b2) based on the microorganism data, determining whether a microorganism characteristic is to be determined; and
- by the electronic processor of the supervisory system, generating a characteristic subset with samples for which it is determined that the microorganism characteristic is to be determined.

It will be appreciated that the and/or construction of the first aspects relates to the steps b1), c1), d1) together, and/or b2).

Compared to the method of WO2021112673, the method according to the first aspect may provide faster results and/or results may be obtained using less resources, by virtue of the supervisory system being able to determine whether a subsequent identification analysis is required, or the first set of analysis data is sufficient to identify no, one, or multiple microorganisms in a particular sample, in particular with sufficient confidence, and/or whether a microorganism characteristic is to be determined. Embodiments of the method are envisioned wherein it is not desired to determine whether a microorganism characteristic is to be determined or wherein it is not desired to determine whether a subsequent identification analysis is required. Compared to the method of WO2021112673, the present aspects may allow for a higher throughput of samples using for example the same or similar equipment, such as the analysis arrangements.

The term "sample" or "biological sample", as used herein, includes reference to a sample from a human body, an animal body, a plant, a laboratory culture, an environmental sample or a food item, a pharmaceutical or chemical product. The food item, pharmaceutical or chemical product may be intended to be devoid of microorganisms or microbial DNA, may be suspected of microorganism presence, and/or a general desire may be to analyse the sample for presence of microorganisms and/or for one or more microorganism characteristics.

The biological samples analysed in the method according to the first aspect are preferably biological samples suspected of comprising one or more microorganisms such as archaea, bacteria, viruses, protozoa, parasites, or fungi, or a combination thereof.

Any biological sample may be a bodily sample of subjects selected from a bodily fluid or exudate, including but not limited to uterine fluid, whole blood, serum, plasma, lymph fluid, mucus, saliva, sputum, stool/faeces, sweat, wound fluid, pus/purulence, gastric content, ascites/ascitic fluid, bile, urine, semen, cerebrospinal fluid/liquor, pleural punctate, joint aspirate, tissue, and breast milk or a bodily sample of subjects in the form of a swab, a biopsy, drain fluid, a lavage, or paper point sample, including but not limited to a sample from the skin, an organ, a tissue, the oral cavity, the urogenital tract, the vaginal tract, the gastrointestinal tract, respiratory tract or pulmonary system, and the cardiovascular system. Any set of samples, or subset of samples, may comprise samples according to any example of a sample disclosed herein, in any combination. Generally the subject is human, although as will be appreciated by those in the art, the subject may be an animal. Thus other animals, including mammals and birds are included within the definition of subject.

In general, any analysis arrangement is arranged for performing one or more analysis steps on one or more samples. When an analysis arrangement is arranged for performing one or more analysis steps on one or more samples, typically, but not necessarily, said analysis arrangement may be arranged for performing a batch analysis on multiple samples. Within a single batch, samples may be processed together or one-by-one. A batch may thus refer to a carrier holding multiple samples being processed in a single batch.

Any analysis arrangement may be specified according to one, more, or all of the following parameters:
- Capacity: representing the number of samples which can be processed in a single batch. For example, the capacity may correspond to the sample carrier, such as a well plate, which can be used with the analysis arrangement, with for example 24 or 96 wells.
- Runtime: the typical and/or mean time required to run an analysis on a single set of samples. The runtime may be in the order of seconds, minutes, or even hours.
- Analysis data: representing the type of analysis data obtainable using the analysis arrangement, for example melting curve data, amplification curve data relating to a shape of a qPCR curve, PCR amplicon length, or DNA sequence data. In general, the analysis data may be in one or more colour channels, for example when the analysis data comprises melting curve or amplification curve data.

Any data may be transferred to a supervisory system using any transfer means, for example a wired or wireless data connection, over an intranet, internet, using a data carrier, any other means of transporting data, or any combination thereof. In general, it will be appreciated that the supervisory system may be at a location near any analysis arrangement, or conceivably remote from any analysis arrangement, for example when the supervisory system comprises a remote computing device, for example a remote server and/or cloud server.

Identifying no, one, or multiple microorganisms in a sample, based on analysis data, may result in no microorganisms identified in the sample, meaning a negative result. Alternatively, identifying no, one, or multiple microorganisms in a sample, based on analysis data, may result in a positive result, *i.e.* determining a presence of one or more microorganisms in the sample, without being able to determine with sufficient confidence at species or strain level which one or multiple specific microorganisms are present in the sample. Preferably, however, identifying no, one, or multiple microorganisms in a sample, based on analysis data, results in the supervisory system determining with sufficient confidence at species or strain level which one or multiple specific microorganisms are present in the sample.

When the required confidence level for identifying no, one, or multiple microorganisms in the sample is not met, the supervisory system can determine that a subsequent identification analysis is required for a particular sample. The subsequent identification analysis results in further analysis data, which further analysis data can be used by the supervisory system to again attempt to identifying no, one, or multiple microorganisms in one or more samples. Based on the further analysis data, it is also possible that the supervisory system determines again that a subsequent identification analysis is required.

Based on identifying no, one, or multiple microorganisms in a sample, microorganism data can be generated by the supervisory system. Such microorganism data can be stored on an electronic memory comprised by the supervisory system, and/or an electronic memory remote from the supervisory system requiring transfer of the microorganism data to the remote memory. Microorganism data can be generated based on any set of analysis data, and microorganism data may be augmented after further analysis data is obtained by the supervisory system.

Determining, by the supervisory system, whether further identification analysis of a sample is required may thus be based on a confidence level determined by the supervisory system. The confidence level is indicative of the confidence with which no, one, or more microorganisms can be identified in the sample. For example, the supervisory system may be instructed to require a 95% confidence level, a 99% confidence level, or any other confidence threshold.

In any method disclosed herein, when the supervisory system identifies no, one, or more microorganisms with a confidence below the required confidence threshold, a preliminary analysis output may be generated by the supervisory system. The preliminary analysis output may for example be indicative of one or more microorganisms being detected, without having sufficient confidence in identifying the exact type or species of microorganisms in the sample.

For any method according to the first aspect, by the electronic processor of the supervisory system, a characteristic subset may be generated with samples for which it is determined that one or more microorganism characteristics is or are to be determined. The electronic processor of the supervisory system may be used to determine whether any microorganism characteristic is to be determined based on the microorganism data.

Microorganism characteristics may for example relate to antimicrobial resistance, strain typing, a virulence factor, strain-specific characteristics to identify a specific clone, factors from which it can be deduced whether a micro-organism is dead or alive, any othe characteristic, or any combination thereof. Microorganism characteristics may be determined using conventional techniques. For example, based on microorganism data indicating that in a number of samples a bacteria is present, it may be determined that antimicrobial resistance is to be determined only for those samples in which a bacteria is present. This may be determined regardless of whether the specific strain of bacteria has been determined yet. An indication of the presence of any bacteria above a particular confidence threshold may be sufficient to add a particular sample to the characteristic subset with samples for which it is determined that the microorganism characteristic is to be determined.

It will be understood that when multiple characteristics can be determined, for each characteristic an individual characteristic subset may be generated.

The determining that a microorganism characteristic is to be determined may be based on the first set of analysis data, and more in particular on the microorganism generated as a result of the step of identifying no, one, or multiple microorganisms in the sample.

For example, when one or more microorganism have been identified in a sample with sufficient confidence, based on the type or strain of microorganism identified, it may be determined that it is required to determine one or more microorganism characteristics for one or multiple microorganisms in the sample. To determine that it is required to determine one or more microorganism characteristics for a particular sample, it may not be required that the exact microorganism is identified. Rather, for example the mere presence of one or more microorganism, or in particular the mere presence of one or more types of microorganism such as bacteria, may trigger that one or more microorganism characteristics should be identified.

It is also conceived that after one or more microorganism have been identified in a sample using the third set of analysis data, it is determined that one or more microorganism characteristics should be determined. It is envisioned that with the third set of analysis data, compared to only using the second set of analysis data, a determination with higher confidence can be made that no, one, or more microorganisms are present in the sample. As such, it is possible that only after obtaining this higher confidence, it is determined that one or more microorganism characteristics should be determined for a sample. Also this sample can be added to a characteristic subset with samples for which it is determined that the microorganism characteristic is to be determined.

In general, any method step can be performed by an electronic processor of the supervisory system, which may be the same electronic processor for each step, or conceivably multiple electronic processors may be used in a single method. As such, whenever "the electronic processor" is referred to, it will be appreciated that multiple electronic processors at the same location or remote from each other may be used.

A second aspect of the present disclosure provides a supervisory system for analysing a set of biological samples. The supervisory system comprises:
- an input for receiving analysis data for the set of biological samples, which analysis data is obtained using an analysis arrangement; and
- an electronic processor, arranged for, based on the analysis data:
   - identifying no, one, or multiple microorganisms in each sample of the set of biological samples and generating microorganism data based on the identifying;
   - determining for each sample in the set of samples whether a subsequent identification analysis is required based on the microorganism data; and
   - generating an identification subset of samples with samples for which it is determined that a subsequent identification analysis is required.

By virtue of the supervisory system, it may be determined which samples in the set of biological samples require a further identification analysis, and for which samples the first set of analysis data is sufficient to identify, with sufficient confidence, no, one, or multiple microorganisms in the sample. Additionally or alternatively, by virtue of the supervisory system, it may be determined for which samples one or more microorganism characteristics are to be determined.

Typically, the first analysis arrangement, the second analysis arrangement, optional third analysis arrangement, and optional further analysis arrangement(s), are stand-alone arrangements unable to communicate or interact with each other, for example unable to transfer data between the arrangements. The supervisory system according to the second aspect now allows analysis data from different analysis arrangements to be used in an efficient manner to increase speed with which microorganisms can be identified in biological samples and/or use less resources to identify microorganisms in biological samples. It will be appreciated that analysis arrangements between which data can be exchanged are also envisioned within the present disclosure.

A third aspect of the present disclosure provides a supervisory system for analysing a set of biological samples, the supervisory system comprising:
- an input for receiving analysis data for the set of biological samples, which analysis data is obtained using an analysis arrangement; and
- an electronic processor, arranged for, based on the analysis data:
   - identifying no, one, or multiple microorganisms in each sample of the set of biological samples and generating microorganism data based on the identifying;
   - determining for each sample in the set of samples whether a microorganism characteristic is to be determined based on the microorganism data; and
   - generating a characteristic subset with samples for which it is determined that the microorganism characteristic is to be determined.

The characteristics subset with samples for which it is determined that the microorganism characteristic is to be determined may for example be visually provided to an operator, such that the operator can perform further steps required for the determination of microorganism characterisics for samples in the characteristics subset. Additionally or alternatively, automated processing steps may be determined, for example by the supervisory controller, based on the characteristics subset with samples.

Any characteristics subset may comprise location data, sample identification data, and/or a particular characteristic which is to be determined.

The electronic processor of any supervisory system, for example according to the second or third aspect, may for example comprise one or more central processing units (CPU) which can be provided with a computer program comprising instructions to cause the electronic processor to execute one or more or all steps of any method according to the first aspect. The present disclosure thus also contemplates a computer-readable medium having stored thereon a computer program comprising instructions to cause the electronic processor to execute one or more or all steps of any method according to the first aspect.

In general, any supervisory system disclosed herein may comprise an electronic processor for generating instructions for automated transfer of one or more samples or parts thereof between locations, for example from a first analysis arrangement to another analysis arrangement. Such instructions may be used to for example control a pipetting robot to transfer samples or parts thereof from one location to another. One location may for example be the first analysis arrangement, and the other location may be the second or third analysis arrangement.

A fourth aspect of the present disclosure provides an analysis system for analysing a set of biological samples, comprising:
- a first analysis arrangement for analysing a set of samples to obtain first analysis data;
- a second analysis arrangement for analysing a subset of samples to obtain first analysis data;
- a supervisory system for analysing the set of biological samples for identification of microorganisms in the samples, in particular according to the second and/or third aspect,
wherein the supervisory system is arranged for receiving first analysis data from the first analysis arrangement, and the supervisory system comprises an electronic processor arranged for, based on the first analysis data, for each sample in the set of samples:
a) identifying no, one, or multiple microorganisms in the sample; or
b) determining that a subsequent identification analysis is required.

Typically, of the analysis arrangements comprised by an analysis system, the first analysis arrangement is preferred in use over the second analysis arrangements, third analysis arrangement, and any further analysis arrangement, by virtue of the first analysis system having a higher capacity, shorter runtime, higher accuracy, being more economic in use, being more readily available, and/or any other advantage over the other analysis arrangements in the analysis system. Similarly, in any method disclosed herein, the runtime of the first analysis arrangement may be shorter than the runtime of the second analysis arrangement and/or the runtime of the second analysis arrangement is shorter than the runtime of the third analysis arrangement

It will be appreciated that features and options disclosed in conjunction with any method disclosed herein may be readily applied to construe a supervisory system according to the second aspect and/or third aspect, and an analysis system according to the fourth aspect.

### BRIEF DESCRIPTION OF THE FIGURES

In the figures,
FIGS 1A-1C schematically show steps in a method of analysing a set of biological samples;
FIG 2 schematically shows an analysis system for analysing a set of biological samples for identification of microorganisms in the samples;
FIGS 3A-3C schematically shows steps in which a single biological sample of a set of biological samples is analysed according to the present disclosure; and
FIG 4 schematically depicts another example of a sample comprised by a set of biological samples, which is analysed for identification of microorganisms potentially present in the sample.

### DETAILED DESCRIPTION OF THE FIGURES

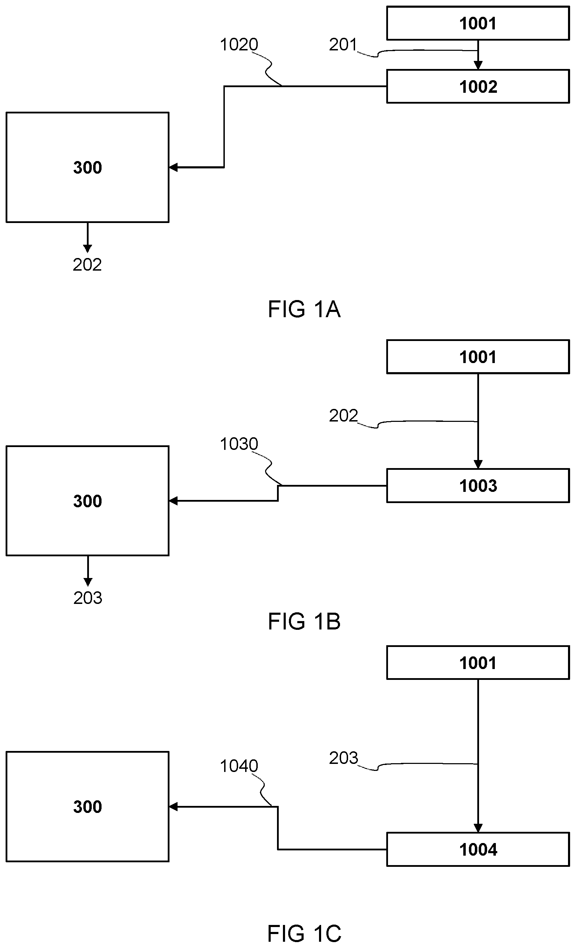

FIGS 1A-1C schematically show steps in a method of analysing a set of biological samples for identification of microorganisms potentially present in the samples, which set may comprise any number of samples. Each sample may be held in an individual container, such as a test tube. All samples in the set of samples may be carried on a single carrier, such as a well plate. Each sample can be identified from sample identification data, for example printed on a label provided on each individual sample container. Preferably, the sample identification data is not readily traceable to a specific subject, but instead represents an alias for a specific subject.

In an optional pre-processing step 1001, any number of pre-processing steps can be applied to the samples in the set of samples in order to prepare the samples for analysis. Pre-processing may for example comprise isolating nucleic acids comprised in the biological samples and/or PCR amplification of the samples to generate PCR amplicons.

After the optional pre-processing, the first set of samples 201 is analysed in a first analysis arrangement in a first analysis step 1002. As a result of the first analysis step 1002, first analysis data 1020 is obtained, which is transferred to supervisory system 300 which supervises the method of analysing the set of samples.

Based on the first analysis data 1020, per sample in the first set of samples 201, the supervisory system 300 determines whether a subsequent identification analysis step is required for each sample in the first set of samples 201. As such, a first identification subset of samples 202 is determined by the supervisory system 300, wherein the first identification subset of samples 202 consists of samples of which it has been determined that a subsequent identification analysis step is required. The samples contained in the first identification subset of samples 201 may all be contained in the first set of samples, but also samples from other sets of samples may be contained in the first subset of samples.

For example, the supervisory system 300 may fill the first identification subset of samples 202 up to a number corresponding to the capacity of the second analysis arrangement. In examples, a second set of samples and/or any further sets of samples have to be analysed in the first analysis arrangement before the number of samples in the first identification subset of samples 202 corresponds to the capacity of the second analysis arrangement. In other examples, the number of samples in the first identification subset of samples 202 is lower than the maximum capacity of the second analysis arrangement when the second analysis arrangement is used to analyse the samples in the first subset of samples 202. The same applied to the second identification subset of samples and the third analysis arrangement, and any further identification subset of samples and any further analysis arrangement which may be comprised by any analysis system.

For every sample in the set of samples 201 for which no, one, or multiple microorganisms have been identified, for example with a confidence level above a particular threshold, a further analysis in a further analysis arrangement may no longer be required. As such, the time spent and/or resources required for identification of microorganisms in these samples may be reduced, for example compared to the method of WO2021112673 where two analyses methods are used for each sample. Additionally or alternatively, when no further analysis of a sample is required after the first analysis step 1002, the capacity of any further analysis arrangement may be used more efficiently for analysis of only those samples for which further analysis is required.

In FIG 1B, steps of the method are depicted wherein the first identification subset of samples 202 is analysed in a second analysis step 1003 in a second analysis arrangement. The second analysis arrangement is preferably arranged for performing a different type of analysis than the first analysis arrangement, and as such different analysis data can be obtained using the second analysis arrangement, compared to the analysis data obtained using the first analysis arrangement. As a result of the second analysis step 1003, second analysis data 1030 is obtained, which is transferred to supervisory system 300 which supervises the method of analysing the set of samples. It will be appreciated that depending on the first analysis data 1020, the first identification subset of samples 202 may be empty and no further analysis of the samples of the first set of samples may be required.

Based on the second analysis data 1030, the supervisory system 300 determines whether a subsequent identification analysis is required for each sample in the first identification subset of samples 202. As such, a second identification subset of samples 203 is determined by the supervisory system 300 containing samples from the first identification subset of samples 202 for which is determined that a further analysis is required, and optionally also containing samples from other sets of samples previous analysed. In examples, the second identification subset of samples 203 may be empty, for example when for each sample in the first subset of samples 202, no, one, or multiple microorganisms have been identified, for example with a sufficient confidence level.

In FIG 1C, steps of the method are depicted wherein the second identification subset of samples 203 is analysed in a third analysis step 1004, in a third analysis arrangement. As a result of the third analysis step 1004, third analysis data 1040 is obtained. The third analysis data 1040 is used by the supervisory system 300 to identify no, one, or multiple microorganisms in the sample or samples in the second subset of samples 203.

The third analysis arrangement is preferably arranged for performing a different type of analysis than the first and second analysis arrangements, and as such different analysis data can be obtained using the third analysis arrangement, compared to the analysis data obtained using the first analysis arrangement and the second analysis arrangement.

In any step of identifying no, one, or multiple microorganisms in a sample, microorganism data can be generated based on the identifying. In general, microorganism data can for each sample be indicative for example of no microorganism being present in the sample, the presence of one or more microorganisms in the sample on a microorganism level (e.g. bacteria or virus), or the presence of a particular strain or species of a particular microorganism (*e.g*. the species E. coli or the strain E. coli O157:H7), or an inconclusive result, all typically with a particular level of confidence.

The present disclosure does not exclude that the same biological sample is analysed two or more times in the same analysis arrangement. For example, if the analysis data from the first time the sample is analysed in a particular analysis arrangement is inconclusive, the supervisory system 300 may determine that a second analysis of the same sample in the same particular analysis arrangement is required.

It will be appreciated that although FIGS 1A-1C depict an example in which a maximum of three different analysis steps 1002, 1003, 1004 are performed, the methods disclosed herein may be readily modified to incorporate any one or more additional steps using any one or more additional analysis arrangements which may be comprised by any analysis system.

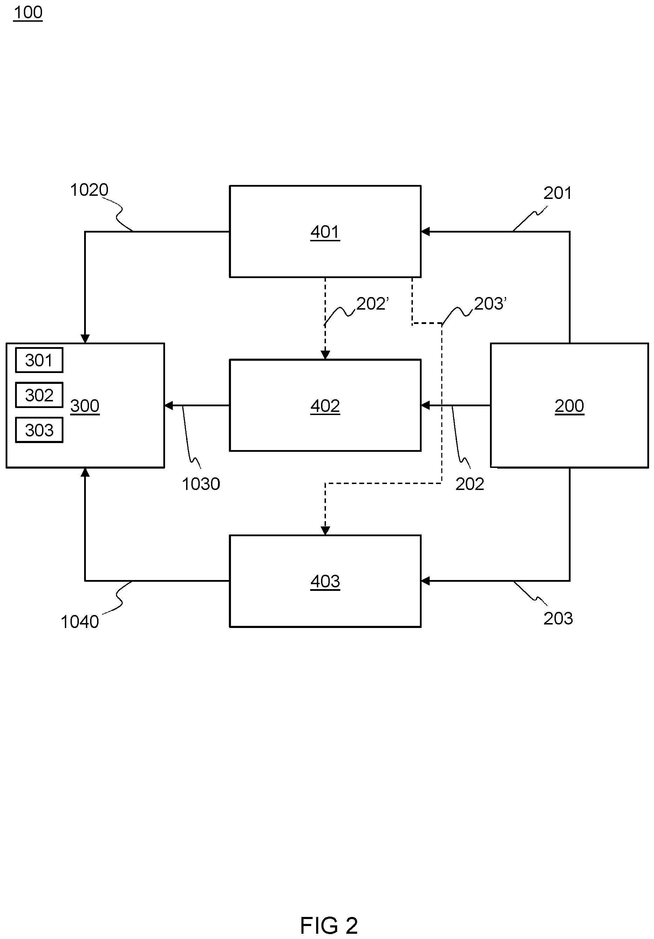

FIG 2 schematically shows an analysis system 100 for analysing a set of biological samples 201 for identification of microorganisms in the samples. The set of samples 201 are for example provided in a carrier 200, which carrier can for example be a be a 96-wells plate. A carrier may also be arranged to only carry a single sample or subsample.

The system 100 as depicted in FIG 2 comprises a first analysis arrangement 401, second analysis arrangement 402, and optional third analysis arrangement 403. The system 100 may be readily modified to include any additional number of analysis arrangements.

Preferably, but not necessarily, the first analysis arrangement 401 is arranged to obtain melting curve data and/or amplification curve data from samples analysed by said first analysis arrangement 401. Melting curve data may for example be based on temperature-dependent dissociation between two DNA-strands as measured using a DNA-intercalating fluorophore. It is also envisioned that the first analysis arrangement 401 is arranged to obtain PCR amplicon length data or amplification curve data, additional or alternative to the melting curve data.

Preferably, but not necessarily, the second analysis arrangement 402 is arranged to obtain PCR amplicon length data. The PCR amplicon length data may for example be recorded by the second analysis arrangement 402 using capillary electrophoresis. It is also envisioned that the second analysis arrangement 401 is arranged to obtain melting curve data and/or amplification curve data.

Preferably, but not necessarily, the third analysis arrangement 403 is arranged to obtain DNA sequence data. The DNA sequence data may be obtained by sequencing DNA present in a sample. It is also envisioned that the second analysis arrangement is arranged to obtain DNA sequence data or amplification curve data, and the third analysis arrangement is omitted.

Any of these preferences, for example related to any analysis arrangement, in any combination, may be readily applied to any method and analysis system disclosed herein.

The supervisory system 300 comprises an electronic processor 301, such as a CPU, an electronic memory 302 on which data can be stored, and an input 303 for receiving data. On the electronic memory 302, which may be directly or remotely accessible for the electronic processor 301, a computer program comprising instructions to cause the electronic processor 301 to execute one or more or all steps of any method disclosed herein may be stored.

The electronic processor 301 may be used for processing analysis data, in order to identify no, one, or multiple microorganisms in a sample, or determine that a subsequent identification analysis is required.

Identifying no, one, or multiple microorganisms in a sample may comprise comparing analysis data associated with said sample with a database comprising comparative analysis data. The database comprising comparative analysis data may be stored on the memory 302 of the supervisory system 300 and/or may be obtained from a remote memory, for example via an intranet or internet connection. Additionally or alternatively, identifying no, one, or multiple microorganisms in a sample may comprise using a trained model to provide a prediction of the presence of no, one, or multiple microorganisms in the sample based on analysis data associated with said sample. The model may for example be a machine learning model, trained using reference analysis data.

Whenever a trained model is used for identifying no, one, or multiple microorganisms in a sample, results obtained using analysis data of a second analysis arrangement may be used to further train the trained model which receives as input analysis data of a first analysis arrangement. This may be advantageous for example when the analysis data obtained by the second analysis arrangement is more accurate for identifying no, one, or multiple microorganisms in a sample than the analysis data obtained by the first analysis arrangement. Similarly, identification results obtained using analysis data of a third analysis arrangement may be used to train the trained model which received as input analysis data of a first analysis arrangement or a second analysis arrangement.

For any method disclosed herein, the original set of samples 201 may be sampled to obtain the samples in the first subset of samples 202 and/or second subset of samples 203, for example using a micropipette operated by a human operated or by an automated system. As such, it may be required, by the supervisory system 300, to track where samples are present in the analysis system 100, for example in which analysis arrangement. Material from the same sample may be simultaneously present in the original set of samples 201, and in one or more subsets of samples, for example in one or more of the analysis arrangements. The automated system may be provided with instructions by the supervisory system on what subsamples to form from which sample, and where in which analysis arrangement the subsamples are to be transported to. For example, a pipetting robot may be used to transport samples between two locations.

It is also envisioned that samples in a subset of samples, such as the first subset of samples 202 and/or second subset of samples 203, are not obtained from the original set of samples 201. Instead, samples in the subset of samples are obtained after the sample has been analysed in for example the first analysis arrangement. In the first analysis arrangement, for example, PCR amplicons are generated. The second analysis arrangement and/or third analysis arrangement may use these PCR amplicons in their analysis to obtain analysis data.

Each sample in the original set of samples 201 may be provided with and/or accompanied with sample identification data, for example as text and/or a machine-readable barcode on a label on a container holding each respective sample. Any method disclosed herein may comprise a step of, by the supervisory system, obtaining sample identification data on each sample in the set of biological samples, and storing the sample identification data in a sample dataset, for example in the memory 302.

Obtaining sample identification data may be an automated process for example using one or more cameras and/or scanners, or require an operator manually providing the sample identification data to the supervisory system 300.

The sample identification data may be associated with a particular location of the sample. Each sample has a location in a carrier 200 in which the set of biological samples 201 is held. The carrier 200 may for example be a 96-well plate, holding 96 or less individual biological samples. The location of each sample of which sample identification data has been obtained may be associated in the sample dataset with a particular location on a particular carrier. In the example of Table 1, the source location is structured A,B,C, with A identifying the carrier, B identifying the column of the respective carrier, and C identifying the row of the respective carrier.

Next to the carrier wherein the original set of samples 201 are held, further carriers may present in the analysis system 100. For example, the first identification subset of samples 202 may be located on a second carrier which is compatible with the second analysis arrangement 402. The second identification subset of samples 203 may be located on a third carrier which is compatible with the third analysis arrangement 403.

In any method of analysing a set of biological samples, a subsample may be obtained from a sample in the set of samples. Such a subsample may be transferred to any analysis arrangement, for analysis. In general, transfer of a subsample may require a human operator, but may also be automated. When a subsample is obtained of a sample, preferably, sufficient volume of the sample remains intact in order for more subsamples to be obtained from the same sample, for example when the sample has to be analysed in multiple analysis arrangements. Conceivably, a subsample may also be transferred to an arrangement for determining one or more microorganism characteristics.

A subsample may also be obtained from an analysis arrangement, where the subsample has been previously processed by the analysis arrangement. For example, in the first analysis arrangement, PCR amplicons may be generated which can also be used in a second and/or third analysis arrangement.

In any method disclosed herein, as an optional feature for any supervisory system, location data of subsamples may be stored in the sample dataset. Location data is indicative of where subsamples are located, for example in an analysis arrangement. Using the location data, analysis data and/or microorganism data obtained using an analysis arrangement may be assigned to the correct sample in the sample dataset, by the supervisory system.

Location data may be obtained as user input. For example, an operator may provide location data to the supervisory system. Additionally or alternatively, location data may be generated by an analysis arrangement based for example on sample identification data accompanying a subsample, for example in the form of a label, text, barcode, or other machine-readable element on, in, or attached to a carrier holding the subsample.

An example of a location dataset is shown in Table 1:

**Table 1. Example of a location dataset**

| SAMPLE ID | SOURCE LOCATION | ANALYSIS ARRANGEMENT 1 | ANALYSIS ARRANGEMENT 2 | ANALYSIS ARRANGEMENT 3 |
|---|---|---|---|---|
| 1 | 1,1,1 | 2,1,1 | 3,1,1 | 4,1,1 |
| 2 | 1,1,2 | 2,1,2 | - | - |
| 3 | 1,1,3 | 2,1,3 | - | - |
| 4 | 1,1,4 | 2,1,4 | 3,1,2 | - |
| ... | | | | |
| 96 | 1,8,12 | 2,8,12 | 3,4,5 | - |

The location dataset is filled with location data of where a sample with a particular SAMPLE ID is located on first carrier, representing the original source of the samples. As a method of analysing a set of biological samples for identification of microorganisms potentially present in the samples according to the present disclosure is executed, the location dataset can be updated by the supervisory system. In the examples, subsamples of the sample with SAMPLE ID are located on a second carrier associated with the first analysis arrangement, on a third carrier associated with the second analysis arrangement, and on a fourth carrier associated with the third analysis arrangement. Subsamples of the sample with SAMPLE ID 3 are for example only associated with the second carrier associated with the first analysis arrangement, because in the example the supervisory system determined that for the sample with SAMPLE ID 3 no subsequent identification analysis in the second analysis arrangement was required.

In the example, the samples in the set of biological samples have been analysed for identification of microorganisms potentially present in the samples, using a method according to the present disclosure.

The sample with SAMPLE ID 1, or at least a subsample thereof, is first analysed in the first analysis arrangement. As a result of this analysis, a first set of analysis data is obtained, and transferred to the supervisory system.

An example of the first set of analysis data is indicated in Table 2:

**Table 2. Example of first set of analysis data**

| **Column/row** | **1** | ... | **8** |
|---|---|---|---|
| **1** | Melting curve 1 | | Melting curve 8 |
| ... | | | |
| **12** | Melting curve 88 | | Melting curve 96 |

The first set of analysis data can be associated to the correct SAMPLE ID using the location data of Table 1. For example, the supervisory system can determine that the sample on location 1,1 in the first set of analysis data corresponds to the sample with SAMPLE ID 1. Based on the particular entry of the first set of analysis data now associated with the sample of SAMPLE ID 1, the supervisory system may determine that a subsequent identification analysis is required, or the supervisory system may identify no, one or multiple microorganisms in the sample with SAMPLE ID 1.

According to the present disclosure, the supervisory system can generate a first identification subset of samples, and include the sample with SAMPLE ID 1 in the first identification subset of samples. Also any further sample for which, based on the first analysis data, the supervisory system determines that a subsequent identification analysis is required, can be included in the first identification subset of samples.

The first identification subset of samples can be outputted by the supervisory system, for example visually to an operator, or as a set of instructions for an automated system for handling samples. The supervisory system may further generate and output location data indicative for where in the second analysis arrangement which sample, or subsample of a sample, should be located and/or where on a carrier associated with the second analysis arrangement which sample, or subsample of a sample, should be located.

After analysing the samples in the first identification subset of biological samples by the second analysis arrangement, a second set of analysis data is obtained. By the electronic processor of the supervisory system, using the second set of analysis data, for each sample in the first subset of samples, it can be determined if a second subsequent identification analysis is required. Subsequently, a second identification subset of samples can be generated by the electronic processor of the supervisory system, with samples for which it is determined that a second subsequent identification analysis is required, based on the second set of analysis data.

The second identification subset of samples can be outputted by the supervisory system, for example visually to an operator, or as a set of instructions for an automated system for handling samples. The supervisory system may further generate and output location data indicative for where in the third analysis arrangement which sample, or subsample of a sample, should be located and/or where on a carrier associated with the third analysis arrangement which sample, or subsample of a sample, should be located.

After analysing the samples in the second identification subset of biological samples, a third set of analysis data is obtained. Using the third set of analysis data, for each sample in the second identification subset of samples, the supervisory system can identify no, one, or multiple microorganisms in the sample. The identified no, one or more multiple microorganisms are associated with the correct SAMPLE ID in the sample dataset by the supervisory system, for example using location data.

An example of a sample dataset acquired using a method of analysing the set of samples in the first analysis arrangement, the first identification subset of samples in the second analysis arrangement, and the second identification subset of samples in the third analysis arrangement is depicted in Table 3. The sample dataset holds microorganism data for each sample.

**Table 3. Sample dataset with microorganism data**

| **SAMPLE ID** | **RESULT 1** | **RESULT 2** | **RESULT 3** |
|---|---|---|---|
| 1 | Inconclusive | Inconclusive | Positive for a, b |
| 2 | Negative | - | - |
| 3 | Positive for x | - | - |
| 4 | Positive, inconclusive | Positive for y | - |
| ... | | | |
| 96 | Inconclusive | Positive for a | - |

Preferably, analyses are performed for each sample in the set of samples until, with sufficient confidence, it has been determined that the sample is negative, or positive for one or more microorganisms, for example on a microorganism, strain or species level. Analysis data, for example in the first or second set of analysis data, may be indicative of the sample being positive for the presence of microorganisms, but the confidence threshold for indicating one or more particular species or strains of microorganisms may not yet be reached. Such a positive indication may be already presented to an operator, for example as a visual output. Based on such a positive indication, for example, it may be determined that a particular microorganism characteristic is to be determined.

Any feature discussed herein in conjunction with the first analysis arrangement may be readily applied to the second, optional third, and optional further analysis arrangements. Any feature discussed herein in conjunction with the first identification subset of samples may be readily applied to the second identification subset of samples or any further identification subset of samples.

As options depicted with dotted lines in Fig. 2, it is envisioned to transfer the first identification subset of samples 202' from the first analysis arrangement 401 to the second analysis arrangement 402 and/or to transfer the second identification subset of samples 203' from the first analysis arrangement 401 to the third analysis arrangement 403. As such, for example, any processing step, such as PCR amplification, performed at the first analysis arrangement 401 and also required at the second analysis arrangement 402 and/or third analysis arrangement 403 only has to be performed at the first analysis arrangement 401. The optional transfers indicated with dotted lines can replace transfer of samples from the carrier 200 to the respective second analysis arrangement 402 and/or third analysis arrangement 403. Transfer of samples from the second analysis arrangement 402 to the third analysis arrangement 403 is also envisioned as an option.

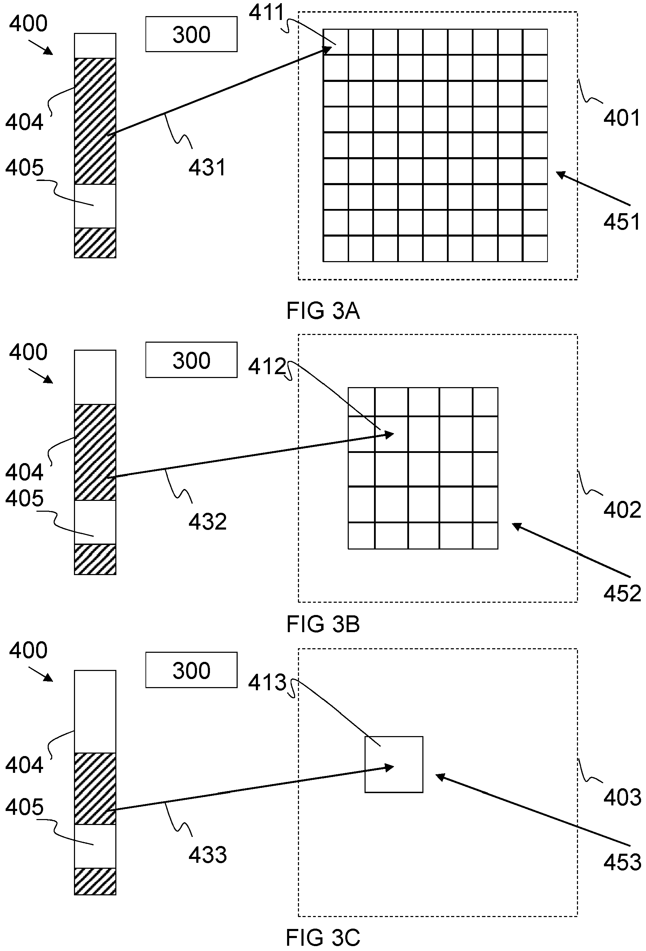

FIGS 3A-3C schematically shows steps in which a single biological sample 400 of a set of biological samples is analysed according to the present disclosure, for example together with other samples in the set of samples which are for clarity and conciseness not depicted in FIGS 3A-3C. The sample 400 can be held on a first carrier, together with the other samples in the set of samples.

The sample 400 is provided in a container 404, which container 404 is provided with sample identification data 405. A first subsample 431 is sampled from the sample 400, and is transferred to a first location 411 of a second carrier 451, associated with the first analysis arrangement 401. The first location 411 may be determined by the supervisory arrangement 300, or the first location 411 may be provided to the supervisory arrangement 300 as part of location data indicative of where subsamples are located.

FIG 3B depicts a situation after the supervisory system 300 has determined that a subsequent identification analysis is required for the sample 400. A second subsample 432 of the sample 400 is transferred to a second location 412 of a third carrier 452, associated with the second analysis arrangement 402. The second location 412 may be determined by the supervisory arrangement 300, or the second location 412 may be provided to the supervisory arrangement 300 as part of location data indicative of where subsamples are located.

FIG 3C depicts a situation after the supervisory system 300 has determined that a further subsequent identification analysis is required for the sample 400. A third subsample 433 of the sample 400 is transferred to a third location 413 of a fourth carrier 453, associated with the third analysis arrangement 403. The third location 413 may be determined by the supervisory arrangement 300, or the third location 413 may be provided to the supervisory arrangement 300 as part of location data indicative of where subsamples are located.

As options schematically indicated in FIGS 3A-3C, the second carrier 451 may have a larger capacity than the third carrier 452, the third carrier 452 may have a larger capacity than the fourth carrier 453, and/or the fourth carrier may have a capacity of a single subsample.

Allthough in the example of Figs. 3B-3C, the second subsample 432 and the third subsample 433 are as an example obtained from the original sample 404, it is also envisioned in any method and analysis system disclosed herein that a subsample is obtained from an analysis arrangement. For example, in the first analysis arrangement, PCR amplicons may be generated in each sample. For the second and/or third analysis arrangement, it may be preferred that the subsample provided to these arrangement has previously been processed in the first analysis arrangement, for example to make use of the generated PCR amplicons.

Next to identifying no, one, or multiple microorganisms in samples, the present disclosure also contemplates identifying one or more microorganism characteristics for one or more microorganisms in samples. Examples of microorganism characteristics are antimicrobial resistance and strain typing. Microorganism data obtained using analysis data, for example from one or more of the first, second, third, and/or further sets of analysis data, can be used by the supervisory system to determine whether one or more microorganism characteristics are to be determined for each sample.

In order for the supervisory system to determine that one or more microorganism characteristics should be determined for a particular sample, microorganism data of the sample is used.

For example, the microorganism data may indicate that a particular microorganism is present in the sample. For example, the presence of bacteria in a sample such as a urine sample may be identified, without necessarily identifying the particular strain of the bacteria. Based on the detected presence of the particular microorganism, the supervisory system may determine that it is required to identify one or more microorganism characteristics.

The supervisory system may be provided with a characteristics dataset in which particular microorganism characteristics are linked to particular strain or species of microorganisms and/or particular types of biological samples. For example, the characteristics dataset may comprise data linking a urinal sample and the presence of bacteria to a need to identify antimicrobial resistance of the bacteria.

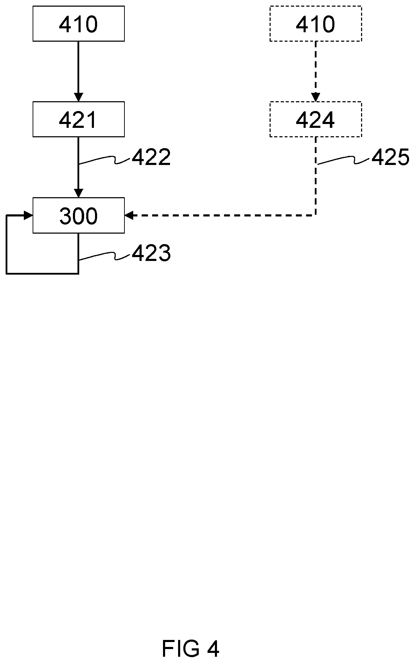

Fig. 4 schematically depicts another example of a sample 410 comprised by a set of biological samples, which is analysed for identification of microorganisms potentially present in the sample. The sample 410 is first analysed in a first analysis arrangement 421. As a result of the analysis, first analysis data 422 on the sample 410 is obtained. The first analysis data 422 may be comprised by a first set of analysis data when multiple samples in a set of samples are analysed in the first analysis arrangement 421. The first analysis data 422 is transferred to a supervisory system 300. Based on the first analysis data 422, an electronic processor of the supervisory system 300 attempts to identify no, one or multiple microorganisms in the sample 410.

In general, for example, the identification can be based on a trained machine learning program and/or by comparing the first analysis data with known analysis data for particular microorganisms. In this process, the supervisory system 300 generates microorganism data 423 which is associated for example to sample identification data indicative of the sample 410 present in a memory of the supervisory system 300.

The present disclosure contemplates that based on the microorganism data 423, the supervisory system 300 determines whether a microorganism characteristic is to be determined - regardless of whether the sample should be analysed in a further analysis arrangement or not.

For example, the microorganism data 423 may be compared with characteristics data in a characteristics dataset, which is for example stored in a memory of the supervisory system 300 or otherwise accessible by the supervisory system. In the characteristics dataset, microorganism characteristics are linked to particular strains or species of microorganisms and/or particular types of biological samples. For example, the characteristics dataset may comprise data linking a urinal sample and the presence of bacteria to a need to identify microbial resistance of the bacteria. The presence of bacteria is an example of microorganism data 423. In another example, the characteristics dataset may comprise data linking a positive indication that E. coli is present in a sample to a need to identify the specific strain as a microorganisms characteristic. Here, the positive indication that E. coli is present in the sample is an example of microorganism data 423.

It will thus be understood that microorganism data 423 may relate to a sample testing negative to the presence of one or more microorganisms, a sample testing positive to the presence of one or more microorganisms, or a sample testing positive to the presence of one or more specific strains of microorganisms. Table 3 shows an example of a sample dataset with microorganism data.

In order to determine whether a microorganism characteristic is to be determined, a sample testing positive to the presence of one or more microorganisms may be sufficient, without requiring sufficient confidence to determine te specific strain and/or species of microorganism. As such, a more efficient and less time-consuming process of obtaining microorganism characteristics may be obtained, compared to a process in which the specific strain and/or species of microorganism has to be determined before deciding to determine a microorganism characteristic.

Fig. 4 shows an optional path of steps wherein the sample 410 is analysed in a second analysis arrangement 424, and second analysis data 425 is generated and transferred to the supervisory system 300.

As a first option, the present disclosure contemplates that the analysis of the sample 410 in the second analysis arrangement 424 may be at least partially parallel in time with the process of obtaining one or more microorganism characteristics for the sample 410.

As a second option, the present disclosure contemplates that based on the first analysis data 422, the supervisory system 300 cannot identify, or at least cannot identify with sufficient confidence the presence of one or more microorganisms in the sample 410. As such, the supervisory system 300 can determine that a subsequent identification analysis is required by the second analysis arrangement 424. The analysis by second analysis arrangement 424 results in second analysis data 425, which is used by the supervisory system 300 to generate further microorganism data 423. Based on the microorganism data 423 now augmented with the further microorganism data, the supervisory system 300 may now determine that a microorganism characteristic is to be determined for the sample 410.

It will be appreciated that situations may occur wherein the supervisory system 300 cannot identify, or at least cannot identify with sufficient confidence, the presence of one or more microorganisms in the sample 410 also with the second analysis data 425. In such cases, third analysis data from a third analysis arrangement may be required.

The sample 410 can be part of a set of biological samples. When the set of samples is processed as a batch in the first analysis, the supervisory system 300 may receive the first analysis data as a batch for all samples in the set of samples. Based on the first analysis data, the supervisory system 300 can identify no, one, or multiple microorganisms in each sample in the set of samples, and generate microorganism data based on the identifying for each sample in the set of samples.

Because any supervisory system 300 disclosed herein may as an option be arranged to determine whether a microorganism characteristic is to be determined based on the microorganism data, the supervisory system 300 can determine for each sample in the set of samples whether a microorganism characteristic is to be determined. Subsequently, the supervisory system 300 can generate a characteristics subset of samples for which a microorganism characteristic or the supervisory system 300 can generate subsets of samples per characteristic which is to be determined. Methods may thus comprise determining one or more microorganism characteristics only for the samples in a subset of samples for which the supervisory system has determined that the microorganism characteristic is to be determined. This may prevent having to attempt to determine microorganism characteristics for each sample, and can thus save time and resources.

Using the characteristics subset of samples, for example samples or subsamples thereof may be transferred to a dedicated arrangement for determining the particular characteristic to be determined.

## Claims

1. Method of analysing a set of biological samples, the method comprising steps of:
- in a first analysis arrangement, analysing the samples in the set of biological samples, thereby obtaining a first set of analysis data;
- transferring the first set of analysis data to a supervisory system;
- by an electronic processor of the supervisory system, using the first set of analysis data, for each sample in the set of biological samples:
a) identifying no, one, or multiple microorganisms in the sample and generating microorganism data based on the identifying;
and at least one of:
b1) based on the microorganism data, determining whether a subsequent identification analysis is required;
- by the electronic processor of the supervisory system, generating a first identification subset of samples with samples for which it is determined that a subsequent identification analysis is required;
- in a second analysis arrangement, analysing the samples in the first identification subset of biological samples, thereby obtaining a second set of analysis data;
- transferring the second set of analysis data to the supervisory system; and
- by the electronic processor of the supervisory system, using the second set of analysis data, for each sample in the first subset of samples:
c1) identifying no, one, or multiple microorganisms in the sample and generating microorganism data based on the identifying; or
d1) determining that another subsequent identification analysis is required;
and/or
b2) based on the microorganism data, determining whether a microorganism characteristic is to be determined; and
- by the electronic processor of the supervisory system, generating a characteristic subset with samples for which it is determined that the microorganism characteristic is to be determined.

2. Method according to claim 1, comprising the steps of:
b1) based on the microorganism data generated based on the identifying of no, one or multiple microorganisms in each sample of the set of biological samples, for each sample in the set of biological samples, determining whether a subsequent identification analysis is required;
- by the electronic processor of the supervisory system, generating the first identification subset of samples with samples for which it is determined that a subsequent identification analysis is required;
- in the second analysis arrangement, analysing the samples in the first identification subset of biological samples, thereby obtaining the second set of analysis data;
- transferring the second set of analysis data to the supervisory system; and
- by the electronic processor of the supervisory system, using the second set of analysis data, for each sample in the first subset of samples:
c1) identifying no, one, or multiple microorganisms in the sample and generating microorganism data based on the identifying; or
d1) determining that another subsequent identification analysis is required.

3. Method according to claim 1 or 2, comprising the steps of:
b2) based on the microorganism data, determining whether a microorganism characteristic is to be determined; and
- by the electronic processor of the supervisory system, generating a characteristic subset with samples for which it is determined that the microorganism characteristic is to be determined.

4. Method according to any of the preceding claims, further comprising:
- by the electronic processor of the supervisory system, generating a second identification subset of samples with samples for which it is determined that a subsequent identification analysis is required, based on the second set of analysis data;
- in a second analysis arrangement, analysing the samples in the second subset of biological samples, thereby obtaining a third set of analysis data; and
- by the electronic processor of the supervisory system, using the third set of analysis data, for each sample in the second subset of samples, identifying no, one, or multiple microorganisms in the sample,
in particular wherein the method further comprises:
- by the supervisory system, obtaining second location data indicative of the locations of the samples in the second analysis arrangement; and
- associating the second analysis data with the sample identification data in the sample dataset based on the second location data.

5. Method according to any of the preceding claims, wherein the first set of analysis data comprises melting curve data or amplification curve data.

6. Method according to any of the preceding claims, wherein the second set of analysis data comprises PCR amplicon length data or amplification curve data.

7. Method according to any of the claims 4-6, to the extent dependent on claim 4, wherein the third set of analysis data comprises DNA sequence data.

8. Method according to any of the preceding claims, wherein the biological samples are biological samples suspected of comprising archaea, bacteria, viruses, protozoa, parasites, or fungi, or a combination thereof and/or wherein the biological samples are bodily samples of subjects selected from a bodily fluid or exudate, including but not limited to uterine fluid, whole blood, serum, plasma, lymph fluid, mucus, saliva, sputum, stool/faeces, sweat, wound fluid, pus/purulence, gastric content, ascites/ascitic fluid, bile, urine, semen, cerebrospinal fluid/liquor, pleural punctate, joint aspirate, tissue, and breast milk and/or bodily samples of subjects in the form of a swab, a biopsy, drain fluid, a lavage, or paper point sample, including but not limited to a sample from the skin, an organ, a tissue, the oral or nasal cavity, the urogenital tract, the vaginal tract, the gastrointestinal tract, respiratory tract or pulmonary system, and the cardiovascular system.

9. Method according to any of the preceding claims, further comprising:
- by the supervisory system, obtaining sample identification data on each sample in the set of biological samples, and storing the sample identification data in a sample dataset;
and/or
- by the supervisory system, obtaining first location data indicative of the locations of the samples in the first analysis arrangement; and
- associating the first analysis data with the sample identification data in the sample dataset based on the first location data.

10. Method according to any of the preceding claims, further comprising:
- by the supervisory system, generating second location data for the samples in the first identification subset of samples, which second location data is indicative for a location of each sample in the first subset of samples in the second analysis arrangement.

11. Supervisory system (300) for analysing a set of biological samples, the supervisory system comprising:
- an input (303) for receiving analysis data for the set of biological samples, which analysis data is obtained using an analysis arrangement; and
- an electronic processor (301), arranged for, based on the analysis data:
- identifying no, one, or multiple microorganisms in each sample of the set of biological samples and generating microorganism data based on the identifying;
- determining for each sample in the set of samples whether a subsequent identification analysis is required based on the microorganism data; and
- generating an identification subset of samples with samples for which it is determined that a subsequent identification analysis is required.

12. Supervisory system (300) for analysing a set of biological samples, the supervisory system comprising:
- an input (303) for receiving analysis data for the set of biological samples, which analysis data is obtained using an analysis arrangement; and
- an electronic processor (301), arranged for, based on the analysis data:
- identifying no, one, or multiple microorganisms in each sample of the set of biological samples and generating microorganism data based on the identifying;
- determining for each sample in the set of samples whether a microorganism characteristic is to be determined based on the microorganism data; and
- generating a characteristic subset with samples for which it is determined that the microorganism characteristic is to be determined.

13. Analysis system (100) for analysing a set of biological samples (201), comprising:
- a first analysis arrangement (401) for analysing a set of samples to obtain first analysis data;
- a second analysis arrangement (402) for analysing a subset of samples (202) to obtain first analysis data;
- a supervisory system (300) for analysing the set of biological samples for identification of microorganisms in the samples, in particular according to claim 11 or 12,
wherein the supervisory system is arranged for receiving first analysis data from the first analysis arrangement, and the supervisory system comprises an electronic processor arranged for, based on the first analysis data, for each sample in the set of samples:
a) identifying no, one, or multiple microorganisms in the sample; or
b) determining that a subsequent identification analysis is required.

14. Analysis system according to claim 13, wherein the first analysis arrangement is arranged for determining melting curve data or amplification curve data as first analysis data, and/or
wherein the second analysis arrangement is arranged for determining PCR amplicon length data amplification curve data as second analysis data, for example wherein the second analysis arrangement comprises a capillary electrophoresis device or wherein the second analysis arrangement is arranged for obtaining DNA sequence data as second analysis data.

15. Analysis system according to any of the claims 13-14, further comprising a third analysis arrangement for analysing a subset of samples (203) to obtain DNA sequence data as third analysis data, and wherein the supervisory system is arranged for receiving the third analysis data, and for identifying no, one, or multiple microorganisms in a sample based on the third analysis data.
